# EUROPÄISCHE PATENTSCHRIFT

(11) **EP 0 863 889 B1**
(45) Veröffentlichungstag und Bekanntmachung des Hinweises auf die Patenterteilung: **06.06.2001**
(21) Anmeldenummer: 96939091.3
(22) Anmeldetag: 18.11.1996
(51) Int. Cl.: C07D 307/08

(54) **VERFAHREN ZUR HYDRIERUNG VON DIHYDROFURANEN ZU TETRAHYDROFURANEN**
PROCESS FOR HYDROGENATING DIHYDROFURANES TO TETRAHYDROFURANES
PROCEDE D'HYDROGENATION DE DIHYDROFURANNES EN TETRAHYDROFURANNES

(30) Priorität: 29.11.1995 DE 19544405
(43) Veröffentlichungstag der Anmeldung: 16.09.1998
(73) Patentinhaber: BASF AKTIENGESELLSCHAFT, 67056 Ludwigshafen (DE)
(72) Erfinder: BRÖCKER, Franz, Josef, D-67061 Ludwigshafen (DE); FISCHER, Rolf, D-69121 Heidelberg (DE); KAIBEL, Gerd, D-68623 Lampertheim (DE); PINKOS, Rolf, D-67098 Bad Dürkheim (DE)
(86) Internationale Anmeldenummer: EP9605071
(87) Internationale Veröffentlichungsnummer: WO9719939

(56) Entgegenhaltungen:
- EP-A- 0 198 435
- EP-B- 0 524 216
- US-A- 4 254 039
- PATENT ABSTRACTS OF JAPAN vol. 14, no. 126 (C-0699), 9.März 1990 & JP 02 002880 A (MITSUBISHI PETROCHEM CO LTD), 8.Januar 1990,

## Beschreibung

Die vorliegende Erfindung betrifft ein verbessertes Verfahren zur katalytischen Hydrierung von 2,5- und 2,3-Dihydrofuran (DHF) mit Wasserstoff zu Tetrahydrofuran (THF).

Nach der Lehre der EP-A 524 216 kann 2,5-Dihydrofuran, welches als Nebenkomponenten 3,4-Epoxy-1-buten und Crotonaldehyd enthält, an Nickel- und Platinkatalysatoren mit Wasserstoff zu THF hydriert werden. Gemäß den Beispielen 1 und 2 werden pro Gramm Nickel 3,6 bzw. 3,7 g THF/h gebildet.

In der US-A 4 254 039 wird die Hydrierung von 2,5-DHF zu THF an einem Palladium-Kohle-Katalysator (5 % Pd auf C) beschrieben. Bei nur 51 % Umsatz werden pro Gramm Palladium etwa 2 g THF/h gebildet.

Die genannten Verfahren haben den Nachteil, daß entweder Vollkatalysatoren aus den Aktivmetallen oder Trägerkatalysatoren verwendet werden. Diese weisen einen hohen Anteil an Aktivmetallen auf, die für den eigentlichen katalytischen Schritt nur teilweise genutzt werden können. Verringert man den teuren Aktivmassenanteil, werden aber die Raum-Zeit-Ausbeute sehr niedrig und das Verfahren somit unwirtschaftlich.

Es bestand die Aufgabe, ein Verfahren zu finden, das bei Verwendung geringer Aktivmassenmengen hohe Raum-Zeit-Ausbeuten für die Hydrierung von DHF zu THF erlaubt. Demgemäß wurde ein verbessertes Verfahren zur katalytischen Hydrierung von Dihydrofuranen zu THF gefunden, das dadurch gekennzeichnet ist, daß man einen Katalysator verwendet, bei dem ein Metall oder mehrere Metalle durch Aufdampfen oder Sputtern auf einem Metalldrahtnetz oder einer Metallfolie als Träger aufgebracht worden sind.

Die erfindungsgemäßen Katalysatoren werden durch Aufdampfen bzw. Aufsputtern der Aktivmassen auf einen folien- bzw. gewebeartigen Metallträger hergestellt. Besonders bewährt haben sich metallische Folien bzw. Gewebe aus Werkstoffen mit den Werkstoffnummern: 1.4767, 1.4401 und 1.4301. Diese metallischen Trägermaterialien werden in der Regel durch oxidative Temperung, vorzugsweise an der Luft, bei Temperaturen von 600 bis 1100°C, vorzugsweise 750 bis 1000°C vorbehandelt und anschließend mit der Aktivmasse beschichtet. Nach der Beschichtung kann eine thermische Formierung an der Luft durchgeführt werden. Zu dieser Formierung kann das beschichtete Trägermaterial bei Temperaturen von 200 bis 800°C, vorzugsweise 300 bis 700°C 0,5 bis 2 Stunden an der Luft erhitzt werden. Das so gefertigte Katalysatormaterial kann anschließend durch Verformung zu Monolithen verarbeitet werden. Nach der Reduktion des Katalysators mit Wasserstoff bei Temperaturen von 20 bis 300°C, vorzugsweise von 20 bis 200°C, die man vorteilhaft im Reaktor durchführt, ist der Katalysator einsatzbereit. Bei Edelmetallkatalysatoren kann die Umsetzung auch direkt, ohne vorherige Aktivierung, angefahren werden.

Die Methoden des Aufdampfens und Aufsputterns von Metallen im Vakuum sind im einzelnen in "Handbook of Thin Film Technology", Maissel und Glang, McGraw Hill, New York, 1970, "Thin Film Processes", J.L. Vossen u. W. Kern, Academie Press N.Y. sowie in der EP-A 198 435 beschrieben.

Als Aktivmasse eignen sich prinzipiell Metalle und Metallkombinationen der metallischen Elemente des Periodensystems, bevorzugt Metalle der I., VII. und VIII. Nebengruppe des Periodensystems der Elemente, z.B. Nickel, Kupfer, Cobalt, Ruthenium, Rhodium, Palladium, Rhenium, Iridium und Platin; besonders bevorzugt ist Palladium.

Die Hydrierung kann bei Temperaturen von 10 bis 250°C, bevorzugt 20 bis 200°C, besonders bevorzugt 30 bis 150°C und einem Wasserstoffdruck von 0,5 bis 300 bar, bevorzugt 0,7 bis 200 bar, besonders bevorzugt 1 bis 100 bar durchgeführt werden.

Die Hydrierung wird zweckmäßig in einer Druckapparatur, beispielsweise in einem Rohrreaktor in der Flüssigphase, entweder in Riesel- oder Sumpffahrweise, oder in der Gasphase durchgeführt.

Der Reaktorzulauf besteht bevorzugt aus reinem 2,5- oder 2,3-DHF oder Mischungen aus beiden, er kann aber auch Beimengungen (bis 5 Gew.-%) wie Crotonaldehyd, Butyraldehyd, Vinyloxiran und Wasser und/oder inerte Verdünnungsmittel (bis 90 Gew.-%) wie THF, Dioxan oder Alkohole wie n-Butanol enthalten.

Die erfindungsgemäße Hydrierung von DHF verläuft hochselektiv. Ein Nebenprodukt, das sich in kleinen Mengen, vornehmlich bei sehr niedrigen Wasserstoffdrücken bildet, ist Furan. Dieses ist jedoch in einfacher Weise destillativ von THF abtrennbar, so daß in einfacher Weise 99,99%iges THF erhalten werden kann.

Dihydrofurane können nach der Lehre der US-A 5 034 545, US-A 5 082 956 oder BE-A 674 652 hergestellt werden.

THF wird als großtechnisches Produkt, z.B. als Lösungsmittel oder Ausgangsstoff für Poly-THF, verwendet.

Das erfindungsgemäße Verfahren ermöglicht Gewichtsverhältnisse von Aktivmasse zu gebildetem THF/h von bis zu 15.000.

### Beispiele:

Alle Angaben zu Zusammensetzungen von Ausgangslösungen oder Produktlösungen erfolgen in Gew.-%.

### Beispiel 1:

Drahtgewebe in Leinenbindung aus dem Werkstoff Nr. 1.4767 mit einer Maschenweite von 0,18 mm und einem Drahtdurchmesser von 0,112 mm wurde 5 h bei 900°C an der Luft erhitzt. Anschließend wurde das so vorbehandelte Trägergewebe in einer Elektronenstrahlbedampfungsanlage beidseitig mit 6 nm Palladium bedampft. Die Schichtdicke wurde mittels eines Schwingquarzes gemessen und die Aufdampfrate mit dem Schwingquarz gesteuert. Die aufgedampfte Palladiummenge betrug 138 mg/m². Aus diesem Katalysatorgewebe wurden monolithische Körper geformt. Dazu wurde mittels einer Zahnradwalze ein Teil des Gewebes gewellt. Dieses gewellte Gewebe wurde mit glattem Gewebe zusammengelegt und aufgewickelt. Man erhielt so monolithische Formkörper, die durch Punktschweißen gefestigt wurden.

### Beispiel 2:

Es wurden zwei Katalysatormonolithe von je 20 cm Höhe und 2 cm Durchmesser gemäß Beispiel 1 aus 0,112 m² Katalysatorgewebe gefertigt und mit einer Gewebedichte von 1,79 m²/l entsprechend 0,247 g Pd/l in einen Rohrreaktor eingebaut. Zunächst wurde der Katalysator mit H₂ bei 150°C 2 Stunden reduziert. Nach dem Abkühlen des Reaktorsystems wurde bei 50°C drucklos 2,5-Dihydrofuran in Sumpffahrweise mit Rückführung zusammen mit Wasserstoff über den Katalysator gepumpt. Die Querschnittsbelastung für 2,5-Dihydrofuran betrug 250m³/m² h und für H₂ 220 m³/m² h. Die Raum-Zeit-Ausbeute betrug 0,34 kg THF/1 Kat. h bzw. 1375 g THF/g Pd h. Die gaschromatographischen Analysen vom Einsatzstoff und Hydrierprodukt wiesen folgende Werte auf:
Edukt: 2,5-DHF: 99,0 %, 2,3-DHF: 0,1 %, THF: 0,85 %, Furan: 0,05 %
Produkt: THF: 98,6 %, Furan: 1,4 %

### Beispiel 3:

Analog Beispiel 2 wurde 2,5-Dihydrofuran in einer Druckapparatur bei 80°C und 20 bar in Sumpffahrweise mit Rückführung hydriert. Die Querschnittsbelastung für 2,5-Dihydrofuran betrug 90 m³/m² h und für H₂ 10 m³/m² h. Die Raum-Zeit-Ausbeute betrug 1,65 kg THF/l Kat. h. Bezogen auf die eingebaute Katalysatorgewebemenge von 2,338 m²/l entsprechend 0,322 g Pd/l erhielt man eine Ausbeute bezogen auf die Aktivmasse, von 5120 g THF/g Pd h. Die gaschromatographischen Analysen vom Einsatzstoff und Hydrierprodukt wiesen folgende Werte auf:
Edukt: 2,5-DHF: 98,99 %, 2,3-DHF: 0,07 %, THF: 1,01 %, Furan: 0,04 %
Produkt: THF: 99,7 %, Puran: 0,3 %

## Patentansprüche

1. Verfahren zur katalytischen Hydrierung von 2,5- und 2,3-Dihydrofuran mit Wasserstoff zu Tetrahydrofuran, dadurch gekennzeichnet, daß man einen Katalysator verwendet, bei dem ein Metall oder mehrere Metalle durch Aufdampfen oder Sputtern auf einem Metalldrahtnetz oder einer Metallfolie als Träger aufgebracht worden sind.

2. Verfahren nach Anspruch 1, dadurch gekennzeichnet, daß man den Katalysator vor seiner Verwendung an der Luft bei erhöhten Temperaturen formiert.

3. Verfahren nach Anspruch 1 oder 2, dadurch gekennzeichnet, daß man metallische Träger mit den Werkstoffnummern 1.4767, 1.4401 oder 1.4301 verwendet.

4. Verfahren nach den Ansprüchen 1 bis 3, dadurch gekennzeichnet, daß man die Träger vor dem Aufbringen der Metalle an der Luft auf 600 bis 1100°C erhitzt.

5. Verfahren nach den Ansprüchen 1 bis 4, dadurch gekennzeichnet, daß man Pd-haltige Katalysatoren verwendet.

## Claims

1. A process for the catalytic hydrogenation of 2,5- and 2,3-dihydrofuran with hydrogen to give tetrahydrofuran, wherein use is made of a catalyst in which a metal or a plurality of metals have been deposited by vapor deposition or sputtering on a metal wire mesh or a metal foil as support.

2. A process as claimed in claim 1, wherein the catalyst is activated in air at elevated temperatures before use.

3. A process as claimed in claim 1 or 2, wherein use is made of metallic supports having the material numbers 1.4767, 1.4401 or 1.4301.

4. A process as claimed in any of claims 1 to 3, wherein the supports are heated in air at from 600 to 1100°C prior to deposition of the metals.

5. A process as claimed in any of claims 1 to 4, wherein Pd-containing catalysts are used.

## Revendications

1. Procédé pour l'hydrogénation catalytique du 2,5- et 2,3-dihydrofuranne avec de l'hydrogène en obtenant le tétrahydrofuranne, caractérisé en ce que l'on utilise un catalyseur dans lequel un métal ou plusieurs métaux ont été déposés sur un treillis métallique ou une feuille métallique, en tant que support, soit par dépôt en phase vapeur soit par pulvérisation cathodique.

2. Procédé selon la revendication 1, caractérisé en ce que l'on façonne le catalyseur à l'air et à des températures élevées avant son utilisation.

3. Procédé selon la revendication 1 ou 2, caractérisé en ce que l'on utilise des supports métalliques avec les numéros de matériau 1.4767, 1.4401 ou 1.430.1.

4. Procédé selon les revendications 1 à 3, caractérisé en ce que l'on chauffe les supports à l'air à des températures de 600 à 1 100°C avant d'y déposer les métaux.

5. Procédé selon les revendications 1 à 4, caractérisé en ce que l'on utilise des catalyseurs contenant du Pd.
